# EUROPEAN PATENT APPLICATION

(11) **EP 4 342 997 A1**
(43) Date of publication of application: **27.03.2024**
(21) Application number: 23199376.7
(22) Date of filing: 25.09.2023
(51) Int. Cl.: C12Q 1/04, A47K 5/12, A47K 5/14

(54) **FLUID DISPENSER FOR COLLECTING AND ANALYZING SKIN MICROBIOTA**

(30) Priority: 26.09.2022 US 202263410074 P
(71) Applicant: OP-Hygiene IP GmbH, 4704 Niederbipp (CH)
(72) Inventor: Ophardt, Heiner, 4422 Ringstrasse 2a Arisdorf (CH); Steltenkamp, Siegfried, 53229 Am Bolzplatz 19 Bonn (DE); Claudinon, Julie, 79108 Reutebachgasse 51 Freiburg (DE)
(74) Representative: Grünecker Patent- und Rechtsanwälte PartG mbB

(57) **Abstract**

A fluid dispenser comprising at least one fluid outlet for dispensing a fluid onto an object; a fluid collector for collecting at least some of the fluid after the fluid has contacted the object; and a fluid analyzer for analyzing at least some of the fluid collected by the fluid collector. The fluid dispenser has at least one feature that is selected to cause at least some of the fluid that contacts the object to fall into the fluid collector. The at least one feature preferably comprises at least one of: a location of the at least one fluid outlet; a guide element that guides an orientation of the object; and a dispensing sequence that includes at least two temporally distinct dispensing events.

## Description

### Related Application

This application claims priority to the 26 September 2022 filing date of U.S. Provisional Patent Application No. 63/410,074, which is incorporated herein by reference.

### Field of the Invention

This invention relates to hand cleaning fluid dispensers, and more particularly to a fluid dispenser that is adapted to collect and analyze fluid after the fluid has contacted a user's hand.

### Background of the Invention

It is known to provide a hand cleaning fluid dispenser with a contaminant sensor, as is disclosed in United States Patent No. 9,437,103 to Ophardt, issued September 6, 2016, which is incorporated herein by reference. The contaminant sensor can be used to detect the presence of pathogens, and to provide warnings when pathogens are detected. As disclosed in United States Patent No. 9,437,103, the contaminant sensor can be located in a drip tray, in order to detect any contaminants present in the fluid collected by the drip tray.

The inventors have appreciated a limitation of the prior art is that, under normal operating conditions, the fluid dispensed from a hand cleaning fluid dispenser onto a user's hand is not collected by the drip tray. Rather, all of the fluid dispensed from the dispenser is usually deposited onto the upward facing palm of the user's hand, and the hand is then removed from the dispenser without any fluid falling into the drip tray. The contaminant sensor is thus unable to analyze the fluid deposited onto the user's hand, and is unable to detect any pathogens that may be present on the user's hand.

### Summary of the Invention

To at least partially overcome some of the disadvantages of previously known devices, systems and methods, in one aspect the present invention provides a fluid dispenser including at least one fluid outlet for dispensing a fluid onto an object; a fluid collector for collecting at least some of the fluid after the fluid has contacted the object; and a fluid analyzer for analyzing at least some of the fluid collected by the fluid collector. The fluid dispenser furthermore has at least one feature that is selected to cause at least some of the fluid that contacts the object to fall into the fluid collector. The at least one feature preferably comprises at least one of the following: a location of the at least one fluid outlet; a guide element that guides an orientation of the object; and a dispensing sequence that includes at least two temporally distinct dispensing events.

The inventors have appreciated that by selecting the location of the at least one fluid outlet; providing a guide element that guides the orientation of the object; and/or providing a dispensing sequence that includes at least two temporally distinct dispensing events, the likelihood of fluid contacting the user's hand and then falling into the fluid collector can be increased. This preferably allows the fluid dispenser to collect and analyze samples from the hands of virtually every user of the dispenser, thereby increasing the amount of data that is collected and improving the ability of the dispenser to detect the presence of pathogens on the hands of users.

For example, in some embodiments of the invention the location of the at least one fluid outlet is selected to increase the likelihood that at least some of the fluid dispensed onto a user's hand will fall into the fluid collector, which may for example be in the form of a drip tray. The fluid dispenser may, for example, have a fluid outlet that dispenses fluid sideways towards the hand, for example from a right side or a left side of the hand. This preferably causes at least some of the fluid that contacts the user's hand to run off of the hand and fall into the fluid collector under the force of gravity, rather than merely pooling on top of the hand. Fluid outlets could also be provided below the hand, dispensing the fluid upwardly onto the hand, such that at least some of the fluid falls back down after contacting the hand under the force of gravity.

In some preferred embodiments, the fluid dispenser has multiple outlets located at different positions. For example, the fluid dispenser could be configured to dispense fluid downwardly from above, sideways from a right side, sideways from a left side, upwards from below, and/or from one or more diagonal positions. The fluid could be dispensed from some or all of the outlets simultaneously, or sequentially. For example, the fluid could be dispensed both from above and from the side simultaneously, with the fluid from above being provided primarily to be collected by the hand for sanitizing the hand, and with the fluid dispensed from the side being provided primarily to collect biological particles present on the hand, and then fall off of the hand and into the fluid collector for analysis.

The fluid could also be dispensed from the different fluid outlets at different times. For example, fluid could be initially dispensed from the side, primarily for the purpose of collecting biological particles for analysis, and then fluid could be dispensed from above, primarily for the purpose of sanitizing the hand.

In some embodiments of the invention, the fluid dispenser includes one or more fluid outlets that can move between a storage position and a dispensing position. The movement of the fluid outlet preferably helps to direct the user's hands to preferred positions for performing a desired hygiene and sample collection procedure. For example, in one preferred embodiment the fluid dispenser includes a fluid dispensing member that can extend forwardly from a rear wall of the dispenser. The fluid dispensing member may, for example, extend forwardly after a user has dispensed an allotment of fluid onto the user's hand from an overhead fluid outlet. The movement of the fluid dispensing member towards the extended position preferably prompts the user to remove their hands from the dispenser, and to rub the user's hands together. The movement of the fluid dispensing member can optionally be accompanied by other prompts, such as audiovisual prompts. Rubbing the user's hands together preferably helps to dislodge biological particles from the user's hands and into the fluid.

When in the extended position, the fluid dispensing member preferably provides two spaces on either side of the fluid dispensing member for the user's hands to be placed in a vertical orientation, with the user's palms facing towards the fluid dispensing member. Preferably, there is not enough space on either side of the fluid dispensing member for the user's hands to be placed in a horizontal orientation, with the user's hands facing upwardly. When the user's hands are placed beside the fluid dispensing member, fluid is preferably sprayed sideways from the fluid dispensing member onto the user's hands. The sideways spraying and the vertical orientation of the user's hands preferably causes at least some of the fluid to drip off of the user's hands and into the fluid collector for analysis.

Optionally, the fluid dispensed from the fluid dispensing member could be the same as the fluid dispensed from the overhead outlet. Alternatively, the fluid dispensed from the fluid dispensing member could be a different fluid. The fluid dispensed from the fluid dispensing member could, for example, be water that is used to rinse the hand cleaning fluid off of the user's hands. Alternatively, the fluid dispensed from the fluid dispensing member could be air, blown onto the user's hands to dry the hands and to direct at least some of the hand cleaning fluid off of the hand and into the fluid collector.

In some embodiments, the fluid dispenser has a guide element that guides the orientation of the user's hand, so as to increase the likelihood of fluid contacting the user's hand and then falling into the fluid collector. For example, the fluid dispenser may have a chamber that has a front opening for receiving the user's hands. The shape of the chamber may be selected to restrict the orientation of the user's hands when inserted into the chamber. For example, the chamber may have a V-like shape that forces the user's hands to be tilted to a diagonal position. Because of the diagonal position of the user's hands, at least some of the fluid that is dispensed onto the user's hands from above preferably runs off of the side of the hand and falls into the fluid collector. The angle of the hands is preferably selected so that at least some fluid falls off of the hands and into the fluid collector, while a sufficient volume of fluid remains on the hands to allow for proper hand sanitizing. In some embodiments of the invention, a sufficient volume of fluid needs to be collected in order for the fluid to be analyzed in the desired manner. In these embodiments of the invention, preferably a sufficient volume of fluid falls off of the hands and into the fluid collector to allow for the desired analysis.

Accordingly, in a first aspect the present invention resides in a fluid dispenser comprising: at least one fluid outlet for dispensing a fluid onto an object; a fluid collector for collecting at least some of the fluid after the fluid has contacted the object; and a fluid analyzer for analyzing at least some of the fluid collected by the fluid collector; wherein the fluid dispenser has at least one feature that is selected to cause at least some of the fluid that contacts the object to fall into the fluid collector; and wherein the at least one feature comprises at least one of: a location of the at least one fluid outlet; a guide element that guides an orientation of the object; and a dispensing sequence that includes at least two temporally distinct dispensing events.

In a second aspect, the present invention resides in a fluid dispenser, which optionally incorporates one or more features of the first aspect, wherein the at least one fluid outlet is for dispensing the fluid onto a body of a user of the fluid dispenser.

In a third aspect, the present invention resides in a fluid dispenser, which optionally incorporates one or more features of one or more of the first and second aspects, wherein the at least one fluid outlet is for dispensing the fluid onto a hand.

In a fourth aspect, the present invention resides in a fluid dispenser, which optionally incorporates one or more features of one or more of the first to third aspects, wherein the fluid dispenser comprises a hand cleaning fluid dispenser.

In a fifth aspect, the present invention resides in a fluid dispenser, which optionally incorporates one or more features of one or more of the first to fourth aspects, wherein the fluid comprises a hand cleaning fluid.

In a sixth aspect, the present invention resides in a fluid dispenser, which optionally incorporates one or more features of one or more of the first to fifth aspects, wherein the fluid comprises a hand sanitizer.

In a seventh aspect, the present invention resides in a fluid dispenser, which optionally incorporates one or more features of one or more of the first to sixth aspects, wherein the at least one feature comprises the location of the at least one fluid outlet.

In an eighth aspect, the present invention resides in a fluid dispenser, which optionally incorporates one or more features of one or more of the first to seventh aspects, wherein the at least one feature comprises a structure of the at least one fluid outlet.

In a ninth aspect, the present invention resides in a fluid dispenser, which optionally incorporates one or more features of one or more of the first to eighth aspects, wherein the at least one feature comprises a flow characteristic of the fluid dispensed from the at least one fluid outlet.

In a tenth aspect, the present invention resides in a fluid dispenser, which optionally incorporates one or more features of one or more of the first to ninth aspects, wherein the at least one feature comprises the guide element that guides the orientation of the object.

In an eleventh aspect, the present invention resides in a fluid dispenser, which optionally incorporates one or more features of one or more of the first to tenth aspects, wherein the at least one feature comprises the dispensing sequence that includes the at least two temporally distinct dispensing events.

In a twelfth aspect, the present invention resides in a fluid dispenser, which optionally incorporates one or more features of one or more of the first to eleventh aspects, wherein the guide element guides a user's hand into an angled position for receiving the fluid dispensed from the at least one fluid outlet; and wherein, when the user's hand is in the angled position, a palm of the user's hand is angled away from an upwards facing horizontal position.

In a thirteenth aspect, the present invention resides in a fluid dispenser, which optionally incorporates one or more features of one or more of the first to twelfth aspects, wherein the angled position is selected so that at least some of the fluid dispensed onto the palm of the user's hand flows off of the palm of the user's hand and into the fluid collector.

In a fourteenth aspect, the present invention resides in a fluid dispenser, which optionally incorporates one or more features of one or more of the first to thirteenth aspects, wherein, when the user's hand is in the angled position, the palm of the user's hand is angled between 15 degrees and 180 degrees from the upwards facing horizontal position.

In a fifteenth aspect, the present invention resides in a fluid dispenser, which optionally incorporates one or more features of one or more of the first to fourteenth aspects, wherein, when the user's hand is in the angled position, the palm of the user's hand is angled between 20 degrees and 95 degrees from the upwards facing horizontal position.

In a sixteenth aspect, the present invention resides in a fluid dispenser, which optionally incorporates one or more features of one or more of the first to fifteenth aspects, wherein, when the user's hand is in the angled position, the palm of the user's hand is angled between 20 degrees and 45 degrees from the upwards facing horizontal position.

In a seventeenth aspect, the present invention resides in a fluid dispenser, which optionally incorporates one or more features of one or more of the first to sixteenth aspects, wherein the guide element comprises a chamber for receiving the user's hand, the chamber having a shape that is selected to restrict the orientation of the user's hand when received by the chamber.

In an eighteenth aspect, the present invention resides in a fluid dispenser, which optionally incorporates one or more features of one or more of the first to seventeenth aspects, wherein the chamber is open forwardly for receiving the user's hand.

In a nineteenth aspect, the present invention resides in a fluid dispenser, which optionally incorporates one or more features of one or more of the first to eighteenth aspects, wherein the chamber is defined at least in part by a first guide wall and a second guide wall; wherein the first guide wall and the second guide wall each extend diagonally when viewed from a front of the fluid dispenser; and wherein the first guide wall is spaced from the second guide wall to provide a space for the user's hand to be placed between the first guide wall and the second guide wall.

In a twentieth aspect, the present invention resides in a fluid dispenser, which optionally incorporates one or more features of one or more of the first to nineteenth aspects, wherein the chamber is further defined at least in part by a third guide wall and a fourth guide wall; wherein the third guide wall and the fourth guide wall each extend diagonally when viewed from the front of the fluid dispenser; and wherein the third guide wall is spaced from the fourth guide wall to provide a second space for a second hand to be placed between the third guide wall and the fourth guide wall.

In a twenty first aspect, the present invention resides in a fluid dispenser, which optionally incorporates one or more features of one or more of the first to twentieth aspects, wherein the chamber is shaped like a V when viewed facing the front of the fluid dispenser.

In a twenty second aspect, the present invention resides in a fluid dispenser, which optionally incorporates one or more features of one or more of the first to twenty first aspects, wherein the at least one fluid outlet comprises a side fluid outlet that is positioned on a right side or a left side of the object.

In a twenty third aspect, the present invention resides in a fluid dispenser, which optionally incorporates one or more features of one or more of the first to twenty second aspects, wherein the at least one fluid outlet comprises a bottom fluid outlet that is positioned below the object.

In a twenty fourth aspect, the present invention resides in a fluid dispenser, which optionally incorporates one or more features of one or more of the first to twenty third aspects, wherein the at least one fluid outlet comprises at least two fluid outlets that are positioned at different locations relative to the object.

In a twenty fifth aspect, the present invention resides in a fluid dispenser, which optionally incorporates one or more features of one or more of the first to twenty fourth aspects, wherein the fluid dispenser comprises a chamber for receiving a user's hand; wherein the chamber has a rear wall; wherein the fluid dispenser has a fluid dispensing member that extends forwardly from the rear wall; and wherein the at least one fluid outlet comprises a member outlet that is positioned on the fluid dispensing member.

In a twenty sixth aspect, the present invention resides in a fluid dispenser, which optionally incorporates one or more features of one or more of the first to twenty sixth aspects, wherein the fluid dispensing member is moveable between a retracted position and an extended position.

In a twenty seventh aspect, the present invention resides in a fluid dispenser, which optionally incorporates one or more features of one or more of the first to twenty sixth aspects, wherein the at least one fluid outlet comprises a chamber outlet; wherein, when the fluid dispensing member is in the retracted position, the chamber outlet is configured to dispense a first fluid onto the user's hand; wherein, when the fluid dispensing member is in the extended position, the member outlet is configured to dispense a second fluid onto the user's hand; and wherein the fluid collected by the fluid collector comprises at least one of the first fluid and the second fluid.

In a twenty eighth aspect, the present invention resides in a fluid dispenser, which optionally incorporates one or more features of one or more of the first to twenty seventh aspects, wherein the first fluid and the second fluid are identical.

In a twenty ninth aspect, the present invention resides in a fluid dispenser, which optionally incorporates one or more features of one or more of the first to twenty eighth aspects, wherein the first fluid and the second fluid are different.

In a thirtieth aspect, the present invention resides in a fluid dispenser, which optionally incorporates one or more features of one or more of the first to twenty ninth aspects, wherein the fluid dispensing member is configured to move from the retracted position to the extended position after the first fluid is dispensed from the chamber outlet.

In a thirty first aspect, the present invention resides in a fluid dispenser, which optionally incorporates one or more features of one or more of the first to thirtieth aspects, wherein the first fluid comprises a hand cleaning fluid; and wherein the fluid dispenser provides at least one prompt for a user to rub the user's hands together after the first fluid has been dispensed onto the user's hand.

In a thirty second aspect, the present invention resides in a fluid dispenser, which optionally incorporates one or more features of one or more of the first to thirty first aspects, wherein the at least one prompt comprises at least one of: a visual prompt, an audio prompt, a video prompt, and an audiovisual prompt.

In a thirty third aspect, the present invention resides in a fluid dispenser, which optionally incorporates one or more features of one or more of the first to thirty second aspects, wherein the at least one prompt encourages the user to remove the user's hands from the chamber.

In a thirty fourth aspect, the present invention resides in a fluid dispenser, which optionally incorporates one or more features of one or more of the first to thirty third aspects, wherein the at least one prompt comprises a movement of the fluid dispensing member from the retracted position to the extended position.

In a thirty fifth aspect, the present invention resides in a fluid dispenser, which optionally incorporates one or more features of one or more of the first to thirty fourth aspects, wherein the member outlet is configured to dispense the second fluid sideways onto the user's hand.

In a thirty sixth aspect, the present invention resides in a fluid dispenser, which optionally incorporates one or more features of one or more of the first to thirty fifth aspects, wherein the member outlet is configured to dispense the second fluid sideways onto the user's hand, so that at least some of the second fluid contacts the user's hand and then falls into the fluid collector.

In a thirty seventh aspect, the present invention resides in a fluid dispenser, which optionally incorporates one or more features of one or more of the first to thirty sixth aspects, wherein the second fluid is selected to at least one of: rinse the first fluid off of the user's hand; moisturize the user's hand; protect the user's hand; clean the user's hand; and dry the user's hand.

In a thirty eighth aspect, the present invention resides in a fluid dispenser, which optionally incorporates one or more features of one or more of the first to thirty seventh aspects, wherein at least one of a flow rate of the fluid; a volume of the fluid; a viscosity of the fluid; a pressure of the fluid; a spray pattern of the fluid; a pulsing of the fluid; and a turbulence of the fluid is selected so that at least some of the fluid that contacts the object falls into the fluid collector.

In a thirty ninth aspect, the present invention resides in a fluid dispenser, which optionally incorporates one or more features of one or more of the first to thirty eighth aspects, wherein the dispensing sequence comprises a first dispensing event, in which a first fluid is dispensed onto the object, and a second dispensing event, in which a second fluid is dispensed onto the object; and wherein the fluid collected by the fluid collector comprises at least one of the first fluid and the second fluid.

In a fortieth aspect, the present invention resides in a fluid dispenser, which optionally incorporates one or more features of one or more of the first to thirty ninth aspects, wherein the at least one fluid outlet comprises a first outlet and a second outlet; wherein the first outlet dispenses the first fluid; and wherein the second outlet dispenses the second fluid.

In a forty first aspect, the present invention resides in a fluid dispenser, which optionally incorporates one or more features of one or more of the first to fortieth aspects, wherein the second outlet is located to a right side, to a left side, or below the object.

In a forty second aspect, the present invention resides in a fluid dispenser, which optionally incorporates one or more features of one or more of the first to forty first aspects, wherein the first outlet is located above the object.

In a forty third aspect, the present invention resides in a fluid dispenser, which optionally incorporates one or more features of one or more of the first to forty second aspects, wherein the second outlet is moveable between a storage position and a dispensing position.

In a forty fourth aspect, the present invention resides in a fluid dispenser, which optionally incorporates one or more features of one or more of the first to forty third aspects, wherein the second outlet is configured to move from the storage position to the dispensing position after the first fluid is dispensed from the first outlet.

In a forty fifth aspect, the present invention resides in a fluid dispenser, which optionally incorporates one or more features of one or more of the first to forty fourth aspects, wherein the first fluid and the second fluid are identical.

In a forty sixth aspect, the present invention resides in a fluid dispenser, which optionally incorporates one or more features of one or more of the first to forty fifth aspects, wherein the first fluid and the second fluid are different.

In a forty seventh aspect, the present invention resides in a fluid dispenser, which optionally incorporates one or more features of one or more of the first to forty sixth aspects, wherein the first fluid is for cleaning the user's hand.

In a forty eighth aspect, the present invention resides in a fluid dispenser, which optionally incorporates one or more features of one or more of the first to forty seventh aspects, wherein the first fluid is for wetting the user's hand.

In a forty ninth aspect, the present invention resides in a fluid dispenser, which optionally incorporates one or more features of one or more of the first to forty eighth aspects, wherein the second fluid is for cleaning the user's hand.

In a fiftieth aspect, the present invention resides in a fluid dispenser, which optionally incorporates one or more features of one or more of the first to forty ninth aspects, wherein the second fluid is for rinsing the user's hand.

In a fifty first aspect, the present invention resides in a fluid dispenser, which optionally incorporates one or more features of one or more of the first to fiftieth aspects, wherein the second fluid is for drying the user's hand.

In a fifty second aspect, the present invention resides in a fluid dispenser, which optionally incorporates one or more features of one or more of the first to fifty first aspects, wherein the second fluid comprises a gaseous fluid.

In a fifty third aspect, the present invention resides in a fluid dispenser, which optionally incorporates one or more features of one or more of the first to fifty second aspects, wherein the second fluid comprises air.

In a fifty fourth aspect, the present invention resides in a fluid dispenser, which optionally incorporates one or more features of one or more of the first to fifty third aspects, wherein the fluid dispenser provides a prompt to rub the user's hands together after the first fluid is dispensed onto the user's hand.

In a fifty fifth aspect, the present invention resides in a fluid dispenser, which optionally incorporates one or more features of one or more of the first to fifty fourth aspects, wherein the first dispensing event is, at least in part, for delivering a hand cleaning product to the user's hand; and wherein the second dispensing event is, at least in part, for collecting a sample of material from the user's hand for analysis by the fluid analyzer.

In a fifty sixth aspect, the present invention resides in a fluid dispenser, which optionally incorporates one or more features of one or more of the first to fifty fifth aspects, wherein the fluid analyzer is configured to detect biological particles collected from the object.

In a fifty seventh aspect, the present invention resides in a fluid dispenser, which optionally incorporates one or more features of one or more of the first to fifty sixth aspects, wherein the fluid analyzer is configured to analyze a microbiota of a user of the fluid dispenser.

In a fifty eighth aspect, the present invention resides in a fluid dispenser, which optionally incorporates one or more features of one or more of the first to fifty seventh aspects, wherein the fluid analyzer is configured to analyze a skin microbiota of the user.

In a fifty ninth aspect, the present invention resides in a fluid dispenser, which optionally incorporates one or more features of one or more of the first to fifty eighth aspects, wherein the fluid dispenser is configured to be operated touchlessly.

In a sixtieth aspect, the present invention resides in a fluid dispenser, which optionally incorporates one or more features of one or more of the first to fifty ninth aspects, wherein the fluid dispenser comprises at least one sensor for touchlessly detecting the object.

In a sixty first aspect, the present invention resides in a fluid dispenser, which optionally incorporates one or more features of one or more of the first to sixtieth aspects, wherein the at least one fluid outlet comprises a fluid outlet that is located diagonally above, to the side, diagonally below, or below the object.

In a sixty second aspect, the present invention resides in a fluid dispenser, which optionally incorporates one or more features of one or more of the first to sixty first aspects, comprising: a fluid outlet for dispensing a fluid onto an object; a fluid collector for collecting at least some of the fluid after the fluid has contacted the object; and a fluid analyzer for analyzing at least some of the fluid collected by the fluid collector; wherein the fluid dispenser is configured to dispense the fluid onto the object such that at least some of the fluid contacts the object and then falls into the fluid collector.

In a sixty third aspect, the present invention resides in a method of operating a fluid dispenser, which optionally incorporates one or more features of one or more of the first to sixty second aspects, the fluid dispenser comprising: at least one fluid outlet for dispensing a fluid onto an object; a fluid collector for collecting at least some of the fluid after the fluid has contacted the object; and a fluid analyzer for analyzing at least some of the fluid collected by the fluid collector; wherein the fluid dispenser has at least one feature that is selected to cause at least some of the fluid that contacts the object to fall into the fluid collector; and wherein the at least one feature comprises at least one of: a location of the at least one fluid outlet; a guide element that guides an orientation of the object; and a dispensing sequence that includes at least two temporally distinct dispensing events; the method comprising: dispensing the fluid onto the object; collecting at least some of the fluid after the fluid has contacted the object; and analyzing at least some of the fluid collected.

In a sixty fourth aspect, the present invention resides in a method of operating a fluid dispenser, which optionally incorporates one or more features of one or more of the first to sixty third aspects, wherein the fluid dispenser comprises the fluid dispenser in accordance with any one of the first to sixty second aspects.

Further aspects of the invention include:
1. A fluid dispenser, which optionally incorporates one or more features of any one or more of the preceding and/or following aspects, comprising: at least one fluid outlet for dispensing a fluid onto an object; a fluid collector for collecting at least some of the fluid after the fluid has contacted the object; and a fluid analyzer for analyzing at least some of the fluid collected by the fluid collector; wherein the fluid dispenser has at least one feature that is selected to cause at least some of the fluid that contacts the object to fall into the fluid collector; and wherein the at least one feature comprises at least one of: a location of the at least one fluid outlet; a guide element that guides an orientation of the object; and a dispensing sequence that includes at least two temporally distinct dispensing events.
2. A fluid dispenser, which optionally incorporates one or more features of any one or more of the preceding and/or following aspects, wherein the fluid dispenser comprises a hand cleaning fluid dispenser and the fluid comprises a hand cleaning fluid.
3. A fluid dispenser, which optionally incorporates one or more features of any one or more of the preceding and/or following aspects, wherein the at least one feature comprises the location of the at least one fluid outlet.
4. A fluid dispenser, which optionally incorporates one or more features of any one or more of the preceding and/or following aspects, wherein the at least one feature comprises the guide element that guides the orientation of the object.
5. A fluid dispenser, which optionally incorporates one or more features of any one or more of the preceding and/or following aspects, wherein the at least one feature comprises the dispensing sequence that includes the at least two temporally distinct dispensing events.
6. A fluid dispenser, which optionally incorporates one or more features of any one or more of the preceding and/or following aspects, wherein the guide element guides a user's hand into an angled position for receiving the fluid dispensed from the at least one fluid outlet; and wherein, when the user's hand is in the angled position, a palm of the user's hand is angled away from an upwards facing horizontal position.
7. A fluid dispenser, which optionally incorporates one or more features of any one or more of the preceding and/or following aspects, wherein the guide element comprises a chamber for receiving the user's hand, the chamber having a shape that is selected to restrict the orientation of the user's hand when received by the chamber.
8. A fluid dispenser, which optionally incorporates one or more features of any one or more of the preceding and/or following aspects, wherein the chamber is open forwardly for receiving the user's hand; wherein the chamber is defined at least in part by a first guide wall and a second guide wall; wherein the first guide wall and the second guide wall each extend diagonally when viewed from a front of the fluid dispenser; wherein the first guide wall is spaced from the second guide wall to provide a space for the user's hand to be placed between the first guide wall and the second guide wall; wherein the chamber is further defined at least in part by a third guide wall and a fourth guide wall; wherein the third guide wall and the fourth guide wall each extend diagonally when viewed from the front of the fluid dispenser; wherein the third guide wall is spaced from the fourth guide wall to provide a second space for a second hand to be placed between the third guide wall and the fourth guide wall; and wherein the chamber is shaped like a V when viewed facing the front of the fluid dispenser.
9. A fluid dispenser, which optionally incorporates one or more features of any one or more of the preceding and/or following aspects, wherein the at least one fluid outlet comprises a fluid outlet that is located diagonally above, to a side, diagonally below, or below the object.
10. A fluid dispenser, which optionally incorporates one or more features of any one or more of the preceding and/or following aspects, wherein the at least one fluid outlet comprises at least two fluid outlets that are positioned at different locations relative to the object.
11. A fluid dispenser, which optionally incorporates one or more features of any one or more of the preceding and/or following aspects, wherein the fluid dispenser comprises a chamber for receiving a user's hand; wherein the chamber has a rear wall; wherein the fluid dispenser has a fluid dispensing member that extends forwardly from the rear wall; wherein the at least one fluid outlet comprises a member outlet that is positioned on the fluid dispensing member; and wherein the fluid dispensing member is moveable between a retracted position and an extended position.
12. A fluid dispenser, which optionally incorporates one or more features of any one or more of the preceding and/or following aspects, wherein the at least one fluid outlet further comprises a chamber outlet; wherein, when the fluid dispensing member is in the retracted position, the chamber outlet is configured to dispense a first fluid onto the user's hand; wherein, when the fluid dispensing member is in the extended position, the member outlet is configured to dispense a second fluid onto the user's hand; wherein the fluid collected by the fluid collector comprises at least one of the first fluid and the second fluid; and wherein the fluid dispensing member is configured to move from the retracted position to the extended position after the first fluid is dispensed from the chamber outlet.
13. A fluid dispenser, which optionally incorporates one or more features of any one or more of the preceding and/or following aspects, wherein the member outlet is configured to dispense the second fluid sideways onto the user's hand, so that at least some of the second fluid contacts the user's hand and then falls into the fluid collector; wherein the second fluid is selected to at least one of: rinse the first fluid off of the user's hand; moisturize the user's hand; protect the user's hand; clean the user's hand; and dry the user's hand.
14. A fluid dispenser, which optionally incorporates one or more features of any one or more of the preceding and/or following aspects, wherein at least one of a flow rate of the fluid; a volume of the fluid; a viscosity of the fluid; a pressure of the fluid; a spray pattern of the fluid; a pulsing of the fluid; and a turbulence of the fluid is selected so that at least some of the fluid that contacts the object falls into the fluid collector.
15. A fluid dispenser, which optionally incorporates one or more features of any one or more of the preceding and/or following aspects, wherein the dispensing sequence comprises a first dispensing event, in which a first fluid is dispensed onto the object, and a second dispensing event, in which a second fluid is dispensed onto the object; wherein the fluid collected by the fluid collector comprises at least one of the first fluid and the second fluid; wherein the at least one fluid outlet comprises a first outlet and a second outlet; wherein the first outlet dispenses the first fluid; wherein the second outlet dispenses the second fluid; wherein the second outlet is located to a right side, to a left side, or below the object; wherein the first outlet is located above the object; wherein the second outlet is moveable between a storage position and a dispensing position; wherein the second outlet is configured to move from the storage position to the dispensing position after the first fluid is dispensed from the first outlet; wherein the fluid dispenser provides a prompt to rub the user's hands together after the first fluid is dispensed onto the user's hand; wherein the first dispensing event is, at least in part, for delivering a hand cleaning product to the user's hand; wherein the second dispensing event is, at least in part, for collecting a sample of material from the user's hand for analysis by the fluid analyzer; and wherein the fluid analyzer is configured to detect biological particles collected from the object.
16. A fluid dispenser, which optionally incorporates one or more features of any one or more of the preceding and/or following aspects, wherein the at least one feature comprises the location of the at least one fluid outlet; wherein the at least one fluid outlet comprises a fluid outlet that is located diagonally above, to a side, diagonally below, or below the object; and wherein the at least one fluid outlet comprises at least two fluid outlets that are positioned at different locations relative to the object.
17. A fluid dispenser, which optionally incorporates one or more features of any one or more of the preceding and/or following aspects, wherein the at least one feature comprises the guide element that guides the orientation of the object; wherein the guide element guides a user's hand into an angled position for receiving the fluid dispensed from the at least one fluid outlet; and wherein, when the user's hand is in the angled position, a palm of the user's hand is angled away from an upwards facing horizontal position.
18. A fluid dispenser, which optionally incorporates one or more features of any one or more of the preceding and/or following aspects, wherein the at least one feature comprises the dispensing sequence that includes the at least two temporally distinct dispensing events; wherein the dispensing sequence comprises a first dispensing event, in which a first fluid is dispensed onto the object, and a second dispensing event, in which a second fluid is dispensed onto the object; wherein the fluid collected by the fluid collector comprises at least one of the first fluid and the second fluid; wherein the first dispensing event is, at least in part, for delivering a hand cleaning product to the user's hand; and wherein the second dispensing event is, at least in part, for collecting a sample of material from the user's hand for analysis by the fluid analyzer.
19. A fluid dispenser, which optionally incorporates one or more features of any one or more of the preceding and/or following aspects, wherein the fluid analyzer is configured to detect biological particles collected from the object; wherein the fluid dispenser is configured to be operated touchlessly; and wherein the second fluid is selected to at least one of: rinse the first fluid off of the user's hand; moisturize the user's hand; protect the user's hand; clean the user's hand; and dry the user's hand.
20. A fluid dispenser, which optionally incorporates one or more features of any one or more of the preceding and/or following aspects, wherein the at least one feature comprises at least three features, and wherein the at least three features comprise: the location of the at least one fluid outlet; the guide element that guides the orientation of the object; and the dispensing sequence that includes the at least two temporally distinct dispensing events.

### Brief Description of the Drawings

Further aspects and advantages of the invention will appear from the following description taken together with the accompanying drawings, in which:
Figure 1 is a perspective view of a hand cleaning fluid dispenser in accordance with a first embodiment of the present invention, with a fluid dispensing member of the fluid dispenser in a retracted position;
Figure 2 is a front view of the fluid dispenser shown in Figure 1, with the fluid dispensing member in the retracted position;
Figure 3 is a perspective view of fluid dispenser shown in Figure 1, with the fluid dispensing member in an extended position;
Figure 4 is a front view of the fluid dispenser shown in Figure 1, with a user's hand shown inserted into a chamber of the dispenser for receiving fluid from a top fluid outlet;
Figure 5 is a front view of the fluid dispenser shown in Figure 1, showing fluid being dispensed onto the user's hand from the top fluid outlet;
Figure 6 is a front view of the fluid dispenser shown in Figure 1, showing the user's hands inserted into the chamber on either side of the extended fluid dispensing member;
Figure 7 is a front view of the fluid dispenser shown in Figure 1, showing fluid being dispensed onto the user's hands from the extended fluid dispensing member;
Figure 8 is a front view of a hand cleaning fluid dispenser in accordance with a second embodiment of the present invention;
Figure 9 is a front view of a hand cleaning fluid dispenser in accordance with a third embodiment of the present invention;
Figure 10 is a front view of the fluid dispenser shown in Figure 9, showing a user's hands inserted into a chamber of the fluid dispenser; and
Figure 11 is a front view of the fluid dispenser shown in Figure 9, showing fluid being dispensed onto the user's hands from top fluid outlets.

### Detailed Description of the Drawings

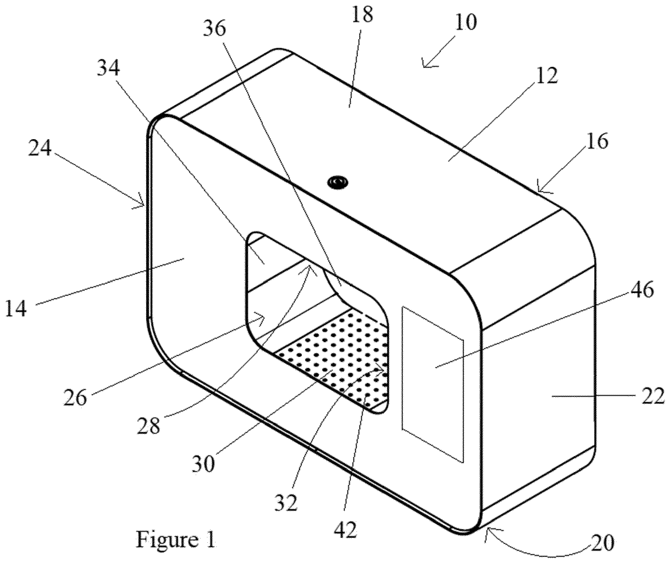

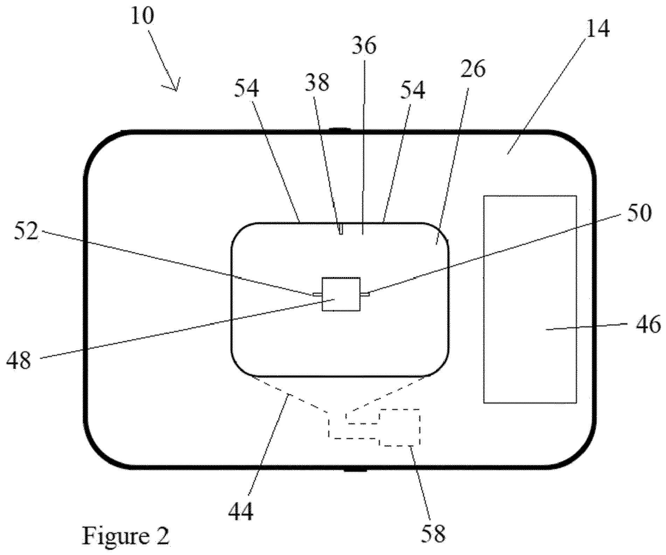

Figures 1 and 2 show a hand cleaning fluid dispenser 10 in accordance with a first embodiment of the present invention. The fluid dispenser 10 has a generally rectangular body 12 with a front wall 14, a back wall 16, a top wall 18, a bottom wall 20, a right side wall 22, and a left side wall 24. The body 12 has a forwardly open chamber 26 that extends rearwardly into the body 12 from the center of the front wall 14.

The chamber 26 is defined by an upper wall 28, a lower wall 30, a right lateral wall 32, a left lateral wall 34, and a rear wall 36. As can be seen in Figure 2, a top fluid outlet 38 extends downwardly from the upper wall 28. Although not visible in the drawings, the body 12 contains a fluid reservoir containing a hand cleaning fluid 40, and a pump mechanism that can dispense the fluid 40 from the top fluid outlet 38 when the pump mechanism is activated. Any suitable fluid reservoir and pump mechanism could be used.

As can be seen in Figure 1, the lower wall 30 has a plurality of apertures 42. The apertures 42 allow fluid 40 that falls onto the lower wall 30 to pass through the lower wall 30 and into a fluid collector 44 that is positioned below the lower wall 30. The fluid collector 44 is shown schematically in Figure 2 by dotted lines. The fluid collector 44 delivers the fluid 40 to a fluid analyzer 58, also shown schematically in Figure 2 by dotted lines.

The fluid analyzer 58 is configured to analyze the fluid 40 to detect contaminants present therein. In preferred embodiments, the fluid analyzer 58 is configured to detect biological particles, such as viruses, bacteria, spores, yeast, and/or fungi. The fluid analyzer 58 may have any structure suitable for analyzing the fluid 40 in order to detect materials, components, particles, and/or contaminants present therein. The fluid analyzer 58 may, for example, have one or more of the structures disclosed in United States Patent No. 9,437,103 to Ophardt, issued September 6, 2016 and United States Patent Application Publication No. 2022/0091011 to Steltenkamp et al., published March 24, 2022, which are incorporated herein by reference.

The front wall 14 of the fluid dispenser 10 has a display 46, which is positioned to the right of the chamber 26. The display 46 may be configured to display any desired information, text, pictures, and/or graphics. For example, the display 46 may be configured to provide visual, video, and/or audiovisual prompts and instructions to assist a user in the operation of the fluid dispenser 10. The display 46 can also be configured to display information such as the results of the fluid analysis, the type of fluid 40 being dispensed, the quantity of fluid 40 being dispensed, and the quantity of fluid 40 remaining in the fluid reservoir. Optionally, the display 46 is a touchscreen that can be touched in order to input commands and information. For example, various settings of the fluid dispenser 10 may be selected or adjusted using the touchscreen display 46, such as the quantity of fluid 40 dispensed with each activation.

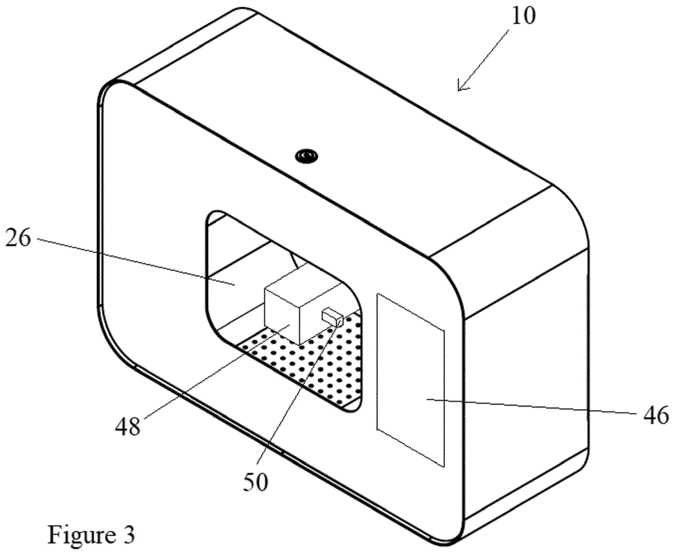

As can be seen in Figures 2 and 3, the fluid dispenser 10 also has a fluid dispensing member 48. The fluid dispensing member 48 is positioned near the center of the rear wall 36 of the chamber 26, and is moveable between a retracted position and an extended position. When in the retracted position, the fluid dispensing member 48 is retracted into the rear wall 36. When in the extended position, the fluid dispensing member 48 extends forwardly from the rear wall 36, as can be seen in Figure 3.

The fluid dispensing member 48 has a right-side fluid outlet 50 that extends sideways to the right and a left-side fluid outlet 52 that extends sideways to the left. Fluid 40 can be dispensed sideways from the right-side fluid outlet 50 and the left-side fluid outlet 52 when the fluid dispensing member 48 is in the extended position. Optionally, the right-side fluid outlet 50 and the left-side fluid outlet 52 are connected to the same fluid reservoir and pump mechanism as the top fluid outlet 38, such that the same fluid 40 is dispensed from each of the outlets 38, 50, 52. Alternatively, the right-side fluid outlet 50 and the left-side fluid outlet 52 could be connected to a different fluid reservoir and/or pump mechanism, and could dispense a different fluid 40 than the top fluid outlet 3 8.

Preferably, the fluid dispenser 10 has one or more hand sensors 54 that are able to detect the presence of a hand 56 or hands 56 inserted into the chamber 26. Any suitable type of sensor 54 capable of detecting the presence of a hand 56 could be used, such as an infrared sensor or a time of flight sensor. The sensor 54 or sensors 54 may be placed at any suitable location for detecting the presence of a hand 56 or hands 56, such as the upper wall 28, the lateral walls 32, 34, or the rear wall 36.

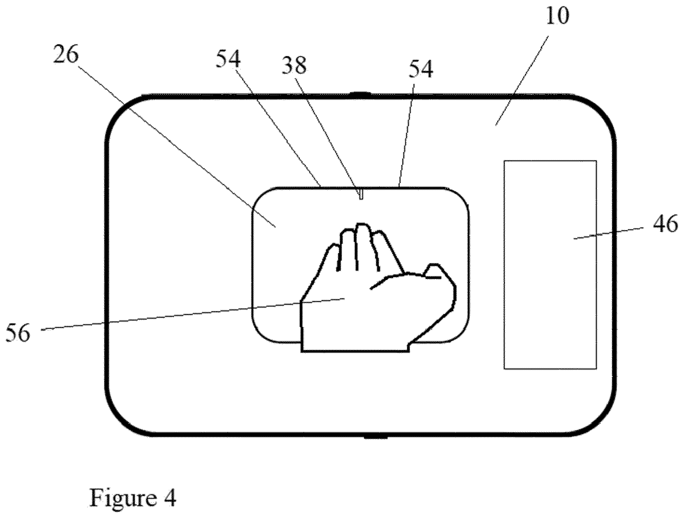

A preferred manner of operating the fluid dispenser 10 will now be described with reference to Figures 1 to 7. When the fluid dispenser 10 is not in use, the fluid dispensing member 48 is preferably maintained in the retracted position. This leaves the chamber 26 unobstructed, with sufficient space for a user to place their hand 56 into the chamber 26 in a horizontal orientation, with the palm of the hand 56 facing upwards, as shown in Figure 4. Optionally, the display 46 may be configured to provide a visual prompt, such as a video, which shows a user how their hand 56 should be positioned in the chamber 26.

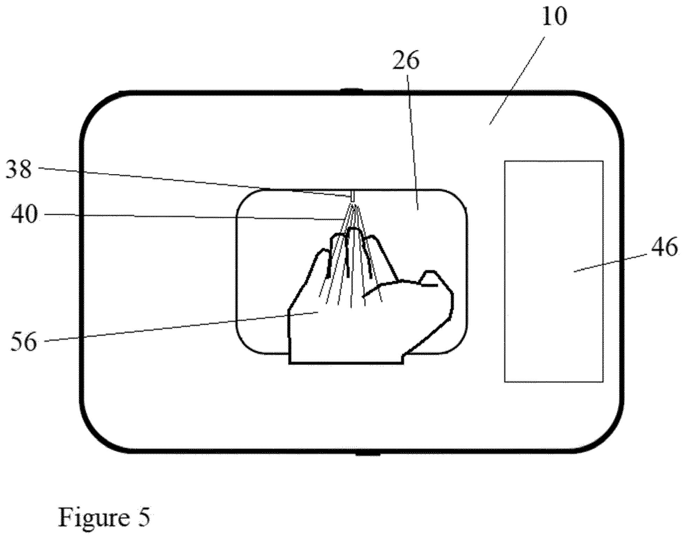

The presence of the user's hand 56 in the chamber 26 is preferably detected by the hand sensors 54, which triggers activation of the pump mechanism. On activation of the pump mechanism, an allotment of fluid 40 is dispensed from the top fluid outlet 38 onto the palm of the user's hand 56, as shown in Figure 5.

Once the fluid 40 has been dispensed onto the user's hand 56, the user preferably removes their hand 56 from the chamber 26 and rubs the user's hands 56 together. This rubbing action spreads the fluid 40 over the user's hands 56, which preferably allows the fluid 40 to disinfect the entire surface area of the hands 56. The rubbing action also preferably helps to dislodge biological particles present on the user's hands 56, such as bacteria and viruses.

Preferably, the fluid dispenser 10 provides one or more prompts encouraging the user to remove their hand 56 from the chamber 26. For example, visual, audio, text, video, and/or audiovisual signals, messages and/or instructions may be provided by the display 46, such as a video showing a user's hands 56 being removed from the chamber 26 and/or rubbed together.

After the fluid 40 has been dispensed onto the user's hand 56 from the top fluid outlet 38, the fluid dispensing member 48 preferably moves from the retracted position to the extended position. The movement of the fluid dispensing member 48 to the extended position also preferably serves as a prompt encouraging the user to remove their hand 56 from the chamber 26. Preferably, the fluid dispensing member 48 moves into the space that is occupied by the hand 56 when the hand 56 is oriented horizontally and positioned underneath the top fluid outlet 38, so as to encourage removal of the hand 56 from the chamber 26.

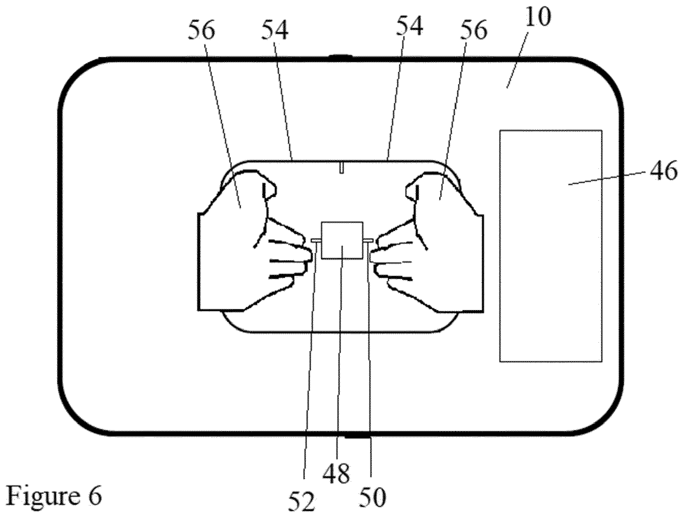

Once the user has had sufficient time to rub their hands 56 together, and the fluid dispensing member 48 has moved into the extended position, the user then preferably places their hands 56 in a vertical orientation on either side of the fluid dispensing member 48, as shown in Figure 6. Preferably, the user is prompted to place their hands 56 in this position, for example by audio and/or visual cues presented on the display 46. The space taken up by the extended fluid dispensing member 48 also encourages the user to place their hands 56 in the vertical orientation.

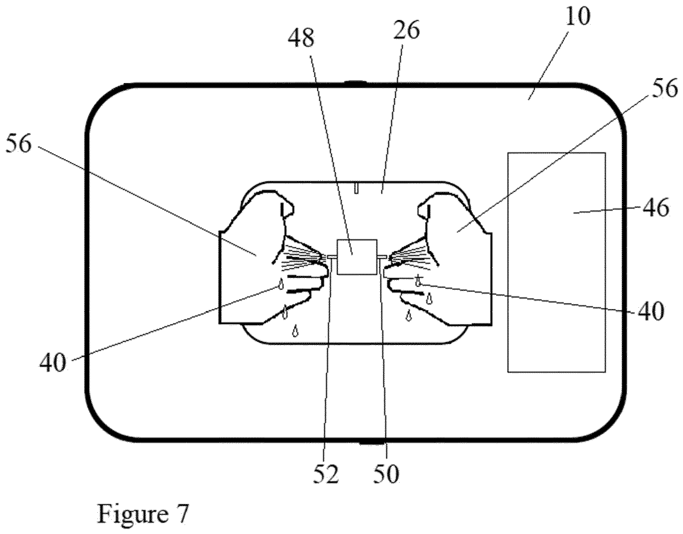

The presence of the user's hands 56 in the chamber 26 is preferably detected by the hand sensors 54, which triggers activation of the pump mechanism and causes fluid 40 to be dispensed sideways onto the palms of the user's hands 56 from the right-side fluid outlet 50 and the left-side fluid outlet 52, as shown in Figure 7. Because the user's hands 56 are oriented vertically, much of the fluid 40 dispensed onto the hands 56 drips off of the hands 56 and into the fluid collector 44.

The fluid 40 that contacts the user's hands 56 and then falls into the fluid collector 44 preferably carries biological particles collected from the user's hands 56. The rubbing of the user's hands 56 together preferably helps to dislodge the particles so that the particles are more easily rinsed off of the hands 56 by the fluid 40. The fluid 40 is then carried to the fluid analyzer 58 for analysis.

The fluid analyzer 58 preferably detects and/or measures contaminants, biological particles, and/or pathogens present in the fluid 40. The fluid analyzer 58 may, for example, be able to detect the presence of specific pathogens, such as disease causing bacteria and/or viruses. The analyzer 58 may also be configured to measure a quantity of biological particles present in the fluid 40. For example, the analyzer may be able to detect when there is a large quantity of bacteria and/or viruses in the fluid 40, without identifying the specific bacteria and/or viruses that are present. A large quantity of bacteria and/or virus particles detected by the analyzer 58 may be interpreted as indicating that the user likely has an infection.

The results of the analysis are preferably stored and/or communicated to an external controlling device or person that collects, analyzes, and interprets the data, preferably a computer, a server, or the like. Preferably, the results obtained from multiple different users over time can provide data that can be used to track and predict the presence of infections in a population and/or in a location over time. This information can be used to inform health and safety policies, such as requiring face masks to be worn in a given location when data from the dispenser 10 or dispensers 10 at that location indicates that individuals with infections are likely present.

Optionally, in some embodiments of the invention, some or all of the results of the analysis may be displayed on the display 46. For example, if the analysis indicates that a user likely has an infection, or that a specific pathogen has been detected, the individual can be instructed to take public health measures such as wearing a mask and/or leaving the facility, and/or undergo further and intensive testing/analysis of the infection.

Optionally, the fluid 40 that is dispensed from the right-side fluid outlet 50 and the left-side fluid outlet 52 may be the same hand cleaning fluid 40 that is dispensed from the top fluid outlet 38. In this case, the fluid 40 from the right-side fluid outlet 50 and the left-side fluid outlet 52 can further clean and/or sanitize the user's hands 56, while also providing for collection and analysis of particles from the user's hands 56.

Alternatively, the fluid 40 dispensed from the right-side fluid outlet 50 and the left-side fluid outlet 52 could be a different fluid 40 provided for a different purpose. For example, the fluid 40 dispensed from the right-side fluid outlet 50 and the left-side fluid outlet 52 could be provided to rinse off the fluid 40 dispensed from the top fluid outlet 38. The fluid 40 dispensed from the right-side fluid outlet 50 and the left-side fluid outlet 52 could, for example, be water. The fluid 40 dispensed from the right-side fluid outlet 50 and the left-side fluid outlet 52 could also be a liquid or solution that is selected to assist with the analysis performed by the fluid analyzer 58, such as a buffer solution. The fluid 40 dispensed from the right-side fluid outlet 50 and the left-side fluid outlet 52 could also provide another function, such as moisturizing the hands 56 or protecting the hands 56 from eczema. In some embodiments of the invention, the fluid 40 dispensed from the right-side fluid outlet 50 and the left-side fluid outlet 52 could be a gaseous fluid such as air, which is blown onto the user's hands 56 in order to dry the hands 56 and to direct some of the hand cleaning fluid 40 present on the user's hands 56 into the fluid collector 44.

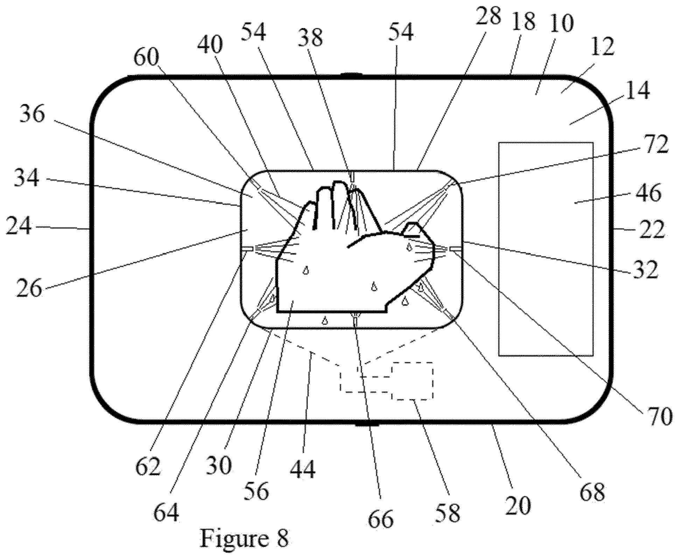

Reference is now made to Figure 8, which shows a fluid dispenser 10 in accordance with a second embodiment of the present invention. Like numerals are used to denote like components.

The fluid dispenser 10 shown in Figure 8 is identical to the dispenser 10 shown in Figures 1 to 7, except that the dispenser 10 lacks the fluid dispensing member 48 and includes a number of additional fluid outlets 60, 62, 64, 66, 68, 70, 72. In particular, in addition to the top fluid outlet 38, the fluid dispenser 10 also has a top left fluid outlet 60, a lateral left fluid outlet 62, a bottom left fluid outlet 64, a bottom fluid outlet 66, and bottom right fluid outlet 68, a lateral right fluid outlet 70, and a top right fluid outlet 72. The lateral left fluid outlet 62 and the lateral right fluid outlet 70 are also sometimes referred to herein as side fluid outlets 62, 70.

The top left fluid outlet 60 is located at the top left corner of the chamber 26, where the upper wall 28 meets the left lateral wall 34, and sprays fluid 40 diagonally downwards and to the right. The lateral left fluid outlet 62 is located on the left side of the chamber 26 on the left lateral wall 34, and sprays fluid 40 sideways to the right. The bottom left fluid outlet 64 is located at the bottom left corner of the chamber 26, where the left lateral wall 34 meets the lower wall 30, and sprays fluid 40 upwards and to the right. The bottom fluid outlet 66 is located on the bottom of the chamber on the lower wall 30, and sprays fluid 40 upwards. The bottom right fluid outlet 68 is located at the bottom right corner of the chamber 26, where the lower wall 30 meets the right lateral wall 32, and sprays fluid 40 upwards and to the left. The lateral right fluid outlet 70 is located on the right side of the chamber 26 on the right lateral wall 32, and sprays fluid 40 sideways to the left. The top right fluid outlet 72 is located at the top right corner of the chamber 26, where the right lateral wall 32 meets the upper wall 28, and sprays fluid 40 diagonally downwards and to the left.

A preferred manner of operating the fluid dispenser 10 will now be described with reference to Figure 8. As in the previous embodiment, the fluid dispenser 10 is touchlessly activated by placing the user's hand 56 into the chamber 26. The hand sensor 54 or hand sensors 54 detect the presence of the user's hand 56, which triggers the activation of a pump mechanism. On activation of the pump mechanism, fluid 40 is dispensed from the fluid outlets 38, 60, 62, 64, 66, 68, 70, 72 onto the hand 56.

By dispensing the fluid 40 from multiple different outlets 38, 60, 62, 64, 66, 68, 70, 72 at different locations, the dispenser 10 is able to increase the likelihood that some of the fluid 40 that contacts the hand 56 will run off of the hand 56 and into the fluid collector 44. Having fluid outlets 60, 62, 64, 66, 68, 70, 72 that dispense the fluid 40 from a side or from below the hand 56 is particularly useful for encouraging fluid 40 to fall off of the hand 56 and into the fluid collector 44. This is because fluid 40 that contacts a side or a bottom of the hand 56 is less likely to pool on top of the hand 56, and is free to fall from the hand 56 under the force of gravity. Having fluid 40 dispensed from above unto the top of the hand 56, usually an upwards facing palm, is particularly effective at cleaning/sanitizing the hand 56, as this allows a volume of the fluid 40 to collect on top of the hand 56 and to be available for the user to rub the fluid 40 into the user's hands 56. Preferably sufficient fluid 40 is dispensed onto the palm of the hand 56 to ensure that the palm is adequately sanitized, as this portion of the hand 56 is most often used to touch surfaces and thus potentially carry and transmit pathogens. More preferably, sufficient fluid 40 is dispensed onto the hand 56 to ensure that the entire hand 56 is adequately sanitized.

By having the fluid 40 dispensed from multiple different locations, the fluid dispenser 10 is able to better ensure that the entire surface of the hand 56 has been adequately sanitized and that at least some fluid 40 will fall off of the hand 56 and into the fluid collector 44 for analysis by the fluid analyzer 58, regardless of the orientation of the hand 56 when the hand 56 is placed in the chamber 26. The fluid analyzer 58 may analyze the fluid 40 in the same ways as in the previous embodiment, and may make use of the resulting data in the same ways as in the previous embodiment.

Optionally, the fluid 40 may be dispensed from all of the fluid outlets 38, 60, 62, 64, 66, 68, 70, 72 simultaneously. Alternatively, fluid 40 could be dispensed from the fluid outlets 38, 60, 62, 64, 66, 68, 70, 72 at different times. For example, fluid 40 could be initially dispensed only from the top fluid outlet 38 in a first dispensing event. The user could then be prompted to remove their hand 56 from the chamber 26 and rub their hands 56 together. The user could then be prompted to place their hand 56 or hands 56 back into the chamber 26, at which time additional fluid 40 would be dispensed from one or more of the remaining outlets 60, 62, 64, 66, 68, 70, 72 onto the hand 56 or hands 56 in a second dispensing event.

Optionally, the fluid 40 dispensed from each of the fluid outlets 38, 60, 62, 64, 66, 68, 70, 72 could be the same or different. In some embodiments of the invention, the fluid 40 dispensed in the first dispensing event may be primarily for sanitizing the user's hands 56, for example, and the fluid 40 dispensed in the second dispensing event may be primarily to rinse off the user's hands 56 and to collect biological particles from the user's hands 56. The first fluid 40 dispensed could be hand sanitizer and the second fluid 40 dispensed could be water, for example.

It is to be appreciated that one or more of the fluid outlets 38, 60, 62, 64, 66, 68, 70, 72 could be omitted and/or could be located at a different position from that shown in Figure 8.

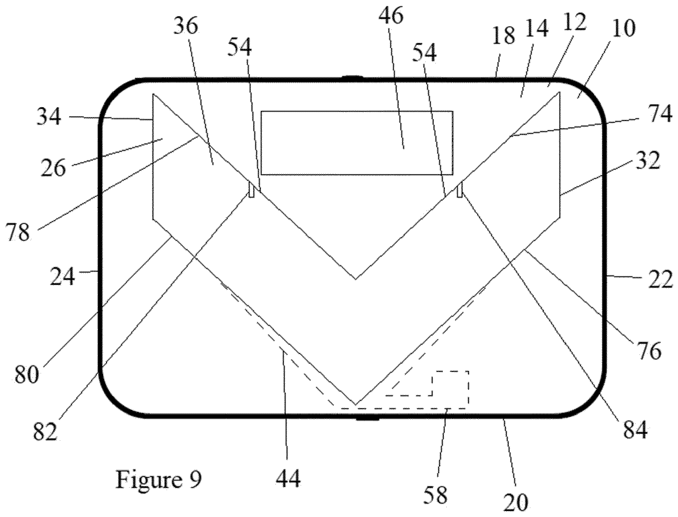

Reference is now made to Figures 9 to 11, which show a fluid dispenser 10 in accordance with a third embodiment of the present invention. Like numerals are used to denote like components.

The fluid dispenser 10 shown in Figures 9 to 11 is generally similar to the fluid dispensers 10 shown in Figures 1 to 8, with the exception that the chamber 26 has a V-like shape rather than a rectangular shape.

The chamber 26 is defined by a right upper wall 74, a right lateral wall 32, a right lower wall 76, a left lower wall 80, a left lateral wall 34, and a left upper wall 78. The right upper wall 74 and the right lower wall 76 are parallel and extend diagonally upwards and to the right. The left upper wall 78 and the left lower wall 80 are parallel and extend diagonally upwards and to the left. The left lateral wall 34 extends vertically between the left upper wall 78 and the left lower wall 80, and the right lateral wall 32 extends vertically between the right upper wall 74 and the right lower wall 76. The right upper wall 74 is also referred to herein as the first guide wall 74; the right lower wall 76 is also referred to herein as the second guide wall 76; the left upper wall 78 is also referred to herein as the third guide wall 78; and the left lower wall 80 is also referred to herein as the fourth guide wall 80.

As can be seen in Figure 9, a left top fluid outlet 82 extends downwardly from the left upper wall 78, and a right top fluid outlet 84 extends downwardly from the right upper wall 74. As in the previous embodiments, the fluid outlets 82, 84 are connected to a fluid reservoir and a pump mechanism for dispensing fluid 40 from the outlets 82, 84.

Although not visible in the drawings, the left lower wall 80 and the right lower wall 76 have apertures 42 similar to those found in the lower wall 30 of the previous embodiments. The apertures 42 allow fluid 40 that falls onto the left lower wall 80 and the right lower wall 76 to pass through the left lower wall 80 and the right lower wall 76 and into a fluid collector 44. As in the previous embodiments, the fluid collector 44 directs the fluid 40 to a fluid analyzer 58 for analyzing the fluid 40.

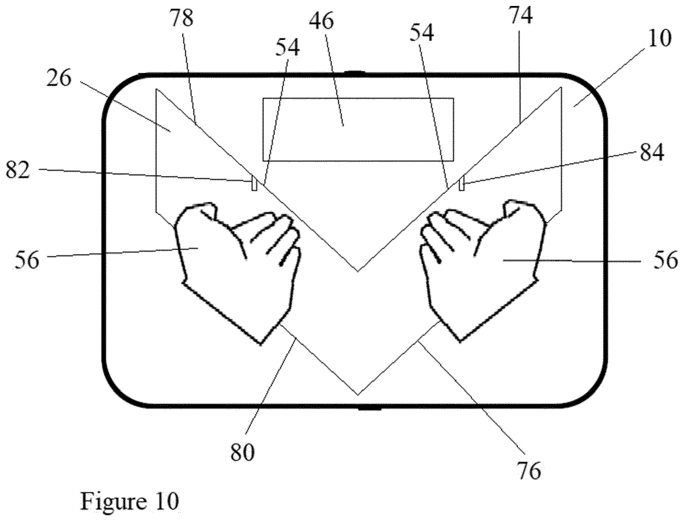

A preferred manner of operating the fluid dispenser 10 will now be described with reference to Figures 9 to 11. As can be seen in Figure 10, the right upper wall 74 and the right lower wall 76 are spaced from each other so as to provide a space for a user to insert their right hand 56 between the right upper wall 74 and the right lower wall 76, under the right top fluid outlet 84. Similarly, the left upper wall 78 and the left lower wall 80 are spaced from each other so as to provide a space for a user to insert their left hand 56 between the left upper wall 78 and the right lower wall 80, under the left top fluid outlet 82.

The V-like shape of the chamber 26 serves to restrict the possible orientations of the user's hands 56 when inserted in the chamber 26, and guides the user's hands 56 to adopt a diagonal orientation, with the right hand 56 being approximately parallel to the right upper wall 74 and the right lower wall 76, and the left hand 56 being approximately parallel to the left upper wall 78 and the left lower wall 80.

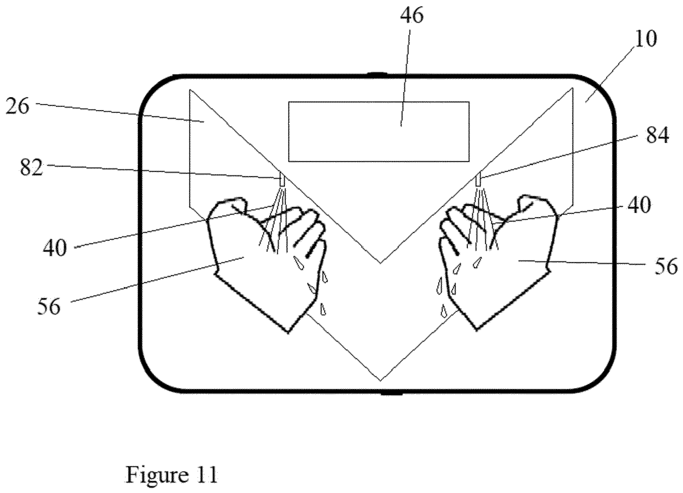

When the user's hands 56 are inserted into the chamber 26, this is preferably detected by the hand sensors 54, which triggers activation of the pump mechanism. On activation of the pump mechanism, the fluid 40 is dispensed from the left top fluid outlet 82 onto the user's left hand 56, and from the right top fluid outlet 84 onto the user's right hand 56, as shown in Figure 11.

The diagonal orientation of the hands 56 preferably allows at least some of the fluid 40 dispensed onto the hands 56 to run off of the hands 56 and into the fluid collector 44 under the force of gravity. The fluid 40 can then be analyzed by the fluid analyzer in the same manner as in the previous embodiments, and the resulting data can be used in the same manner as in the previous embodiments.

Preferably, the user's hands 56 are guided to an orientation in which at least some of the fluid 40 runs off of the hands 56 and into the fluid collector 44, while a sufficient volume of fluid 40 remains on the user's hands 56 to allow for proper cleaning/sanitizing of the user's hands 56.

The chamber 26 could have any desired shape that guides the user's hands 56 to the preferred orientation, and need not have the particular V-like shape shown in the drawings. For example, in an alternative embodiment the dispenser 10 could have separate right and left chambers 26 for receiving each of the left and right hands 56, with each chamber 26 being shaped to encourage the user's hands 56 to be positioned at a preferred orientation, such as vertical or diagonal, with palms facing upwards, sideways, diagonally, and/or downwards.

It is also to be appreciated that other structures could be used to guide the orientation of the user's hands 56, instead of or in addition to the shape of the chamber 56. For example, the fluid dispenser 10 could have a fluid dispensing member 48 similar to that shown in Figure 3, or another obstacle, which may be moveable or immoveable, which restricts or guides the user's hands 56 to a preferred orientation. The feature or features that are used to guide the orientation of the user's hand 56 are generically referred to herein as a guide element.

In some embodiments of the invention, the user's hand 56 is guided to adopt an orientation that is angled away from the horizontal, palm facing upwards position shown in Figure 4. The angle is preferably selected to encourage at least some fluid 40 that contacts the hand 56 to fall into the fluid collector 44. The hand 56 may, for example, be guided to an angled position that is angled between 15 degrees and 180 degrees from the upwards facing horizontal position; between 20 degrees and 95 degrees from the upwards facing horizontal position; between 20 degrees and 45 degrees from the upwards facing horizontal position; or between 30 degrees and 45 degrees from the upwards facing horizontal position. The upwards facing horizontal position may also be referred to as the 0 degree position, and the vertical position with the palm facing sideways may also be referred to as the 90 degree position.

It is to be appreciated that the embodiment shown in Figures 9 to 11 could include any of the features described with reference to the embodiments shown in Figures 1 to 8, and that these features have not been described again merely to avoid repetition, and not because any of the features are exclusive to one or another of the embodiments. For example, the embodiment shown in Figures 9 to 11 could include additional fluid outlets for dispensing fluid 40 from different angles; could be configured to dispense fluid 40 from different outlets at different times; and could be configured to dispense different kinds of fluids 40 from different outlets. Any and all features described with reference to one embodiment of the invention could be included in any of the other embodiments.

It will be understood that, although various features of the invention have been described with respect to one or another of the embodiments of the invention, the various features and embodiments of the invention may be combined or used in conjunction with other features and embodiments of the invention as described and illustrated herein.

The invention is not limited to the particular structures that have been shown in the preferred embodiments. For example, the fluid dispenser 10 need not have a rectangular body 12, with a forwardly open chamber 26. Rather, any suitable structure could be used that allows fluid 40 to contact an object and then be collected for analysis. For example, the fluid dispenser 10 could have a structure similar to those disclosed in United States Patent No. 8,245,877 to Ophardt, issued August 21, 2012; United States Patent No. 8,113,388 to Ophardt et al., issued February 14, 2012; United States Patent No. 8,091,739 to Ophardt et al., issued January 10, 2012; United States Patent No. 7,748,573 to Anhuf et al., issued July 6, 2010; U.S. Patent No. 7,984,825 to Ophardt et al., issued July 26, 2011; U.S. Patent No. 8,684,236 to Ophardt, issued April 1, 2014; U.S. Patent No. 5,373,970 to Ophardt, issued December 20, 1994; U.S. Patent No. 5,836,482 to Ophardt et al., issued November 17, 1998; and/or U.S. Patent No. 9,682,390 to Ophardt et al., issued June 20, 2017, which are each incorporated herein by reference.

In some embodiments of the invention, the chamber 26 may have a depth of about 13 cm, a width of about 25 cm, and a height of about 16 cm.

The fluid dispensing member 48 need not have the particular structure shown in the drawings. For example, the fluid dispensing member 48 could extend downwardly from the upper wall 28, or upwardly from the lower wall 30. The fluid dispensing member 48 could also be positioned outside of the chamber 26 when in a storage position, and pivot and/or extend towards or into the chamber 26 when moved to a dispensing position. Fluid 40 could be dispensed from the fluid dispensing member 48 in any desired direction, such as downwardly, upwardly, diagonally, or to the side.

Preferably, the fluid dispenser 10 is operated touchlessly, though this is not strictly necessary. Any suitable structure for providing touchless operation could be used, such as those described in U.S. 7,984,825 to Ophardt et al., issued July 26, 2011; U.S. 8,397,949 to Ophardt, issued March 19, 2013; U.S. 9,027,788 to Ophardt et al., issued May 12, 2015; U.S. 8,622,243 to Ophardt et al., issued January 7, 2014; U.S. 8,733,596 to Ophardt et al., issued May 27, 2004; and/or U.S. 7,455,197 to Ophardt, issued November 25, 2008, which are incorporated herein by reference.

The fluid dispenser 10 need not include a display 46. The fluid dispenser 10 could also use different mechanisms for providing usage instructions and/or prompts to a user, such as drawings, written instructions, animations, lights, and/or audio alerts.

The fluid outlets 38 need not have the structure shown in the drawings, and need not extend outwardly from the walls of the chamber 26. Preferably, the structure of the outlets 38 and/or the pump mechanism is selected to encourage the fluid 40 to contact the user's hands 56 and then fall into the fluid collector 44. For example, the fluid outlets 38 may be configured to dispense the fluid 40 in a spray pattern that is wide enough that the fluid 40 lands on both the center of the user's palm, where it can collect, as well as the sides of the palm, where it can run off the hand 56 and into the fluid collector 44. The same or a similar effect could also be achieved using a system with multiple nozzles/outlets 38. The fluid dispenser 10 could have more or fewer fluid outlets 38 than is shown in the drawings, and the fluid outlets 38 could have a different location, structure, and orientation than is shown in the drawings.

The fluid pressure may also be selected to increase the likelihood that fluid 40 will contact the hand 56 and then fall into the fluid collector 44. For example, at high pressures the fluid 40 will be more likely to spray off of the hand 56, rather than just pooling on the hand 56. The fluid pressure may, for example, be in the range of 5 psi to 120 psi, or 20 psi to 100 psi. High fluid pressure may also assist in dislodging particles from the user's skin, so that they can be collected and analyzed.

The volume of fluid 40 is also preferably selected to encourage fluid 40 to fall from the hand 56 into the fluid collector 44, while sufficient fluid 40 remains on the hand 56 to allow for proper sanitizing. The volume of fluid 40 dispensed may, for example, be in the range of 0.1 ml to 15 ml. In some embodiments, the volume of fluid 40 dispensed may be between 1.5 ml and 3 ml, or 1.5 ml, or 3 ml, or 1.5 ml per nozzle, or 3 ml per nozzle, or 0.5 ml per nozzle, or 3 ml per pump activation, or 1.5 ml per pump activation, or 0.5 ml per pump activation. In some embodiments, the duration of a dispensing event may be between 0.1 seconds and 10 seconds, or between 0.5 seconds and 3 seconds.

The type of fluid 40 that is dispensed may also be selected to encourage fluid 40 falling off the hand 56 and into the fluid collector 44. For example, fluids 40 having a lower viscosity may be selected because they will more easily flow off of the hand 56 under the force of gravity.

Other fluid flow characteristics may also be selected to encourage the fluid 40 to fall from the hand 56 and into the fluid collector 44, such as dispensing the fluid 40 in pulses, with a turbulent flow, and/or with an aerated flow. The spray duration, spray pattern, nozzle geometry, and location may also be optimized. In some embodiments, the outlets 38 preferably provide a high fluidic ablation force on the palm of the hand 56, which preferably helps to remove biological particles from the skin, and enough volume of fluid 56 to allow a sufficient sample size for analysis. Different nozzle shapes, as well as tubing diameter and length can also be optimized and adjusted. For example, some embodiments of the invention may employ regular tube openings, tubes with circular or other shaped cross-sections, jet-stream nozzles, laminarisator nozzles, shower-type nozzles, spray nozzles, and/or nozzles that provide a variety of different flow velocities and pressures, and any combination thereof.

Although the preferred embodiments have shown the invention as being used to clean and collect samples from a user's hands 56, the invention could also be used to clean and/or collect samples from other objects and body parts. For example, the invention could be used to dispense fluid 40 onto a user's feet, body, and/or head. The invention could also be used to dispense fluid 40 onto an object such as a toothbrush, a wipe, a towel, a sponge, a tool, clothing, and/or any other object from which a sample for analysis might be desired.

Preferably, the invention is used in a system that collects data from many fluid dispensers 10 installed at different locations, and uses the data to track and predict infections in a population and/or location over time.

The invention is not limited to any particular construction of the fluid collector 44. Rather any structure that suitably collects fluid 40 and delivers the fluid 40 for analysis may be used. Likewise, the invention is not limited to any particular structure of the fluid analyzer 58. Rather, any structure capable of performing the desired analysis could be used. For example, the fluid analyzer 58 may optionally incorporate a processor, a memory, a camera, a microscope, a microfluidic particle sorter, a filter, an electrode, an impedance measurement device, and/or a chemical sensor. In some embodiments of the invention, the fluid collector 44 may be in the form of a drip tray. In some embodiments of the invention, the fluid analyzer 58 may be able to identify contaminants, such as dead or alive pathogens, or other biological particles.

In preferred embodiments, the invention is incorporated into a hand cleaning fluid dispenser 10 that dispenses a hand cleaning fluid 40. This advantageously allows for the simultaneous cleaning of a user's hands 56 and the collection of data regarding the microbiota, biological particles, pathogens, and/or contaminants present on the user's hands 56. However, in other embodiments of the invention the fluid 40 need not be a cleaning fluid 40, and could instead be a fluid 40 that has another purpose, such as a buffer solution for collecting biological particles and maintaining a constant pH during the analysis of the fluid 40. In some embodiments of the invention, the fluid 40 that contacts the user's hand 56 and then falls off of the hand 56 may be referred to as overspray.

Preferably, the process of cleaning/sanitizing the user's hands 56 and collecting a sample for analysis is completely touchless, with no physical contact between the hands 56 and the dispenser 10. This helps to avoid the spread of pathogens between users of the dispenser 10. In some embodiments of the invention, the user is encouraged to rub their hands 56 together during an intermediate step, which provides mechanical ablation that is helpful for removing biological particles from the skin. Again, the mechanical ablation preferably occurs without any physical contact between the hands 56 and the dispenser 10.

In some embodiments of the invention, the dispenser 10 may be configured to dispense fluid 40 onto the back of a user's hand 56, in addition to or in place of dispensing onto the palm of the hand 56. This feature may be incorporated into any of the embodiments described herein. Optionally, a volume of 0 ml to 3 ml, or 1.5 ml, can be dispensed onto the back of the hand 56, which may assist in disinfecting the hand 56. Preferably at least some fluid 40 is dispensed onto the palm of the hand 56. In some embodiments, 1 ml to 10 ml of fluid 40 is dispensed onto the palm of the hand 56.

In some embodiments of the invention, the hand hygiene event is divided into at least three steps: first a dispensing step; second a mechanical ablation step; and third a rinsing step. In the dispensing step, fluid 40 is dispensed onto the user's hand 56, or optionally onto another object. In the mechanical ablation step, the user's hands 56 are rubbed together, or optionally rubbed against another object such as a cloth or a sponge. In the rinsing step, additional fluid 40 is dispensed onto the hand 56 or object. Preferably at least some of the fluid 40 that contacts the hand 56 or object is collected by the fluid collector 44 during the rinsing step. These three steps could be incorporated into any of the embodiments of the invention described herein.

The fluid outlets 50, 52 located on the fluid dispensing member 48 are sometimes generically referred to herein as member fluid outlets 50, 52. The fluid outlets 38, 60, 62, 64, 66, 68, 70, 72 that are not located on the fluid dispensing member 48 are sometimes generically referred to herein as chamber fluid outlets 38, 60, 62, 64, 66, 68, 70, 72.

On occasion, the terms "first fluid" and "second fluid" are used herein. These terms are used to distinguish between fluids that are dispensed from different outlets and/or at different times. The "first fluid" and the "second fluid" may be the same fluid or different fluids.

In some embodiments of the invention, dispensing fluid 40 from a location other than directly above a user's hand 56, such as from diagonally above, from the side, from diagonally below, or from below, will preferably cause the user to intuitively tilt the user's hand 56 so that the palm faces towards the source of the fluid 40. The resulting angled position of the hand advantageously increases the likelihood that fluid 40 will contact the user's palm and then fall off of the hand 56 and into the fluid collector 44.

The word "cleaning" as used herein includes, but is not limited to, disinfecting and sanitizing.

In embodiments of the invention in which a gas is dispensed, any suitable gaseous fluid could be used, such as air, oxygen, or any mixture of gases. Gaseous fluids may be dispensed from any suitable outlet and at any suitable time. For example, in embodiments in which different fluids are dispensed at different times, the first fluid could be a gaseous fluid, the second fluid could be a gaseous fluid, and/or any subsequent fluid could be a gaseous fluid.

Although this disclosure has described and illustrated certain preferred embodiments of the invention, it is to be understood that the invention is not restricted to these particular embodiments. Rather, the invention includes all embodiments which are functional, electrical, or mechanical equivalents of the specific embodiments and features that have been described and illustrated herein.

## Claims

1. A fluid dispenser (10) comprising:
at least one fluid outlet for dispensing a fluid (40) onto an object;
a fluid collector (44) for collecting at least some of the fluid (40) after the fluid (40) has contacted the object; and
a fluid analyzer (58) for analyzing at least some of the fluid (40) collected by the fluid collector (44);
wherein the fluid dispenser (10) has at least one feature that is selected to cause at least some of the fluid (40) that contacts the object to fall into the fluid collector (44); and
wherein the at least one feature comprises at least one of:
a location of the at least one fluid outlet;
a guide element that guides an orientation of the object; and
a dispensing sequence that includes at least two temporally distinct dispensing events.

2. The fluid dispenser (10) according to claim 1, wherein the fluid dispenser (10) comprises a hand cleaning fluid dispenser (10) and the fluid (40) comprises a hand cleaning fluid (40).

3. The fluid dispenser (10) according to claim 1 or claim 2, wherein the at least one feature comprises the location of the at least one fluid outlet.

4. The fluid dispenser (10) according to any one of claims 1 to 3, wherein the at least one feature comprises the guide element that guides the orientation of the object.

5. The fluid dispenser (10) according to any one of claims 1 to 4, wherein the at least one feature comprises the dispensing sequence that includes the at least two temporally distinct dispensing events.

6. The fluid dispenser (10) according to any one of claims 1 to 5, wherein the guide element guides a user's hand (56) into an angled position for receiving the fluid (40) dispensed from the at least one fluid outlet; and
wherein, when the user's hand (56) is in the angled position, a palm of the user's hand (56) is angled away from an upwards facing horizontal position.

7. The fluid dispenser (10) according to any one of claims 1 to 6, wherein the guide element comprises a chamber (26) for receiving the user's hand (56), the chamber (26) having a shape that is selected to restrict the orientation of the user's hand (56) when received by the chamber (26).

8. The fluid dispenser (10) according to claim 7, wherein the chamber (26) is open forwardly for receiving the user's hand (56);
wherein the chamber (26) is defined at least in part by a first guide wall (74) and a second guide wall (76);
wherein the first guide wall (74) and the second guide wall (76) each extend diagonally when viewed from a front of the fluid dispenser (10);
wherein the first guide wall (74) is spaced from the second guide wall (76) to provide a space for the user's hand (56) to be placed between the first guide wall (74) and the second guide wall (76);
wherein the chamber (26) is further defined at least in part by a third guide wall and a fourth guide wall;
wherein the third guide wall (78) and the fourth guide wall (80) each extend diagonally when viewed from the front of the fluid dispenser (10);
wherein the third guide wall (78) is spaced from the fourth guide wall (80) to provide a second space for a second hand (56) to be placed between the third guide wall (78) and the fourth guide wall (80); and
wherein the chamber (26) is shaped like a V when viewed facing the front of the fluid dispenser (10).

9. The fluid dispenser (10) according to any one of claims 1 to 8, wherein the at least one fluid outlet comprises a fluid outlet that is located diagonally above, to a side, diagonally below, or below the object.

10. The fluid dispenser (10) according to any one of claims 1 to 9, wherein the at least one fluid outlet comprises at least two fluid outlets that are positioned at different locations relative to the object.

11. The fluid dispenser (10) according to any one of claim 1 to 6, wherein the fluid dispenser (10) comprises a chamber (26) for receiving a user's hand (56);
wherein the chamber (26) has a rear wall (36);
wherein the fluid dispenser (10) has a fluid dispensing member (48) that extends forwardly from the rear wall (36);
wherein the at least one fluid outlet comprises a member outlet that is positioned on the fluid dispensing member (48); and
wherein the fluid dispensing member (48) is moveable between a retracted position and an extended position.

12. The fluid dispenser (10) according to claim 11, wherein the at least one fluid outlet further comprises a chamber outlet;
wherein, when the fluid dispensing member (48) is in the retracted position, the chamber outlet is configured to dispense a first fluid (40) onto the user's hand (56);
wherein, when the fluid dispensing member (48) is in the extended position, the member outlet is configured to dispense a second fluid (40) onto the user's hand (56);
wherein the fluid (40) collected by the fluid collector (44) comprises at least one of the first fluid (40) and the second fluid (40); and
wherein the fluid dispensing member (48) is configured to move from the retracted position to the extended position after the first fluid (40) is dispensed from the chamber outlet.

13. The fluid dispenser (10) according to claim 12, wherein the member outlet is configured to dispense the second fluid (40) sideways onto the user's hand (56), so that at least some of the second fluid (40) contacts the user's hand (56) and then falls into the fluid collector (44);
wherein the second fluid (40) is selected to at least one of:
rinse the first fluid (40) off of the user's hand (56);
moisturize the user's hand (56);
protect the user's hand (56);
clean the user's hand (56); and
dry the user's hand (56).

14. The fluid dispenser (10) according to any one of claims 1 to 11, wherein at least one of a flow rate of the fluid (40); a volume of the fluid (40); a viscosity of the fluid (40); a pressure of the fluid (40); a spray pattern of the fluid (40); a pulsing of the fluid (40); and a turbulence of the fluid (40) is selected so that at least some of the fluid (40) that contacts the object falls into the fluid collector (44).

15. The fluid dispenser (10) according to any one of claims 1 to 11, wherein the dispensing sequence comprises a first dispensing event, in which a first fluid (40) is dispensed onto the object, and a second dispensing event, in which a second fluid (40) is dispensed onto the object;
wherein the fluid (40) collected by the fluid collector (44) comprises at least one of the first fluid (40) and the second fluid (40);
wherein the at least one fluid outlet comprises a first outlet and a second outlet;
wherein the first outlet dispenses the first fluid (40);
wherein the second outlet dispenses the second fluid (40);
wherein the second outlet is located to a right side, to a left side, or below the object; wherein the first outlet is located above the object;
wherein the second outlet is moveable between a storage position and a dispensing position;
wherein the second outlet is configured to move from the storage position to the dispensing position after the first fluid (40) is dispensed from the first outlet;
wherein the fluid dispenser (10) provides a prompt to rub the user's hands (56) together after the first fluid (40) is dispensed onto the user's hand (56);
wherein the first dispensing event is, at least in part, for delivering a hand cleaning product to the user's hand (56);
wherein the second dispensing event is, at least in part, for collecting a sample of material from the user's hand (56) for analysis by the fluid analyzer (58); and
wherein the fluid analyzer (58) is configured to detect biological particles collected from the object.
